# EUROPEAN PATENT APPLICATION

(11) **EP 1 970 423 A1**
(43) Date of publication of application: **17.09.2008**
(21) Application number: 07462002.2
(22) Date of filing: 13.03.2007
(51) Int. Cl.: C09K 11/80, H01J 61/00

(54) **Fluorescent lamp for stimulating previtamin D3 production**

(71) Applicant: LightTech Lámpatechnológia Kft., 2120 Dunakeszi (HU)
(72) Inventor: Reich, Lajos, 1141 Budapest (HU); Hess, Mária, 2120 Dunakeszi (HU); Fözöné, Siklósi Zita, 1044 Budapest (HU); Imre, Krisztián, 2600 Vác (HU)
(74) Representative: Mak, Andras

(57) **Abstract**

The invention relates to a fluorescent lamp, preferably a low pressure mercury discharge lamp for stimulating previtamin D3 production. The lamp has a discharge tube with a discharge gas filling. The inside wall of the discharge tube is covered with a phosphor coating for converting short wave UV radiation of the ionized discharge gas into longer wave UV radiation. The discharge tube is closed at both ends and provided with electrodes which are held and lead through a base cap at both ends. The base caps of the lamp are provided with contact pins for connecting the lamp to an electrical power supply.

According to the improvement of the invention the UV light radiating coating of the discharge tube comprises
- a first phosphor for emitting light waves in the UVB spectrum and
- a second phosphor for emitting light waves in the visible light spectrum and for suppressing the emitted light in the UVB spectrum.

## Description

### Technical Field

The invention relates to a fluorescent lamp for stimulating previtamin D3 production. More specifically, to a low-pressure mercury discharge lamp for producing ultraviolet light in a spectrum and with intensity appropriate for stimulating D3 previtamin production in human skin.

### Background of the invention

Vitamin D is a fat-soluble vitamin that is found in food and can also be made in the human body after exposure to ultraviolet (UV) rays from the sun. Sunshine is a significant source of vitamin D because UV rays from sunlight trigger vitamin D synthesis in the skin.

Vitamin D exists in several forms (D1 to D5), each with a different level of activity. The natural form of vitamin D produced in skin when exposed to sunlight is cholecalciferol (D3). It is the result of a reaction between 7-dehydrocholesterol (7-DHC) and UVB ultraviolet light with wavelengths from 290 to 315 nm. This UV spectrum can be found in natural sunlight when the sun is high enough above the horizon for UVB to penetrate the atmosphere. Cholecalciferol is hydroxylated in the liver to 25-hydroxycholecalciferol (25(OH)D₃ or calcidiol) and stored until it is needed. Measuring the blood's calcidiol level is the only way to determine vitamin D deficiency; levels should be between 40 and 60 ng/mL (100 to 150 nMol/L) for optimal health. Calcidiol is further hydroxylated to 1,25-dihydroxycholecalciferol (1,25(OH)2D₃ or calcitriol in the kidneys and in tissues. It is the most potent steroid hormone derived from cholecalciferol and has significant anti-cancer activity. Calcitriol, also referred to as active vitamin D, functions as a hormone because it sends a message to the intestines to increase the absorption of calcium and phosphorus that may be used in the bones. Without vitamin D, bones can become thin, brittle, or misshapen. Vitamin D sufficiency prevents rickets in children and osteomalacia in adults, which are both skeletal diseases that weaken bones. Vitamin D deficiency also contributes to osteoporosis by reducing calcium absorption. Vitamin D malnutrition may possibly be linked to chronic diseases such as cancer (breast, ovarian, colon, prostate, lung, skin and probably a dozen more types), chronic pain, weakness, chronic fatigue, autoimmune diseases like multiple sclerosis and Type 1 diabetes, high blood pressure, mental illnesses (depression, seasonal affective disorder and possibly schizophrenia) heart disease, rheumatoid arthritis, psoriasis, tuberculosis, periodontal disease and inflammatory bowel disease.

According to current studies one needs about 4,000 units of cholecalciferol a day to meet the body's need for vitamin D. Four thousand units of cholecalciferol are equal to 100 micrograms, or 0,1 milligrams. These studies have also shown that 15 to 20 minutes daily exposure to sunlight with an impart angle above 50 degrees can contribute to sufficient previtamin D3 production in the skin.

Season, geographic latitude, part of the day, cloud coverage, smog, and sunscreen affect UV ray exposure and vitamin D synthesis. Insufficient exposure to sunlight causes vitamin D deficiency, which hinders calcium absorption in bones and weakens the immune system. In order to compensate for vitamin D deficiency, vitamin D preparates can be taken in prescribed amounts. As vitamin D is a highly potent vitamin, there is a danger of toxication if it is taken in overdoses.

On the other hand, natural sunlight may be replaced by artificial sun tanning lamps emitting ultraviolet light. When considering the effects of UV radiation on human health and the environment, the range of UV wavelengths is often subdivided into UVA (400-315 nm), also called Long Wave or "blacklight"; UVB (315-280 nm), also called Medium Wave; and UVC (< 280 nm), also called Short Wave or "germicidal".

Conventional sun tanning lamps such as disclosed in US 4,194,125 emit ultraviolet radiation primarily or exclusively in the UVA range and emit no radiation in the UVC range. The usual UVB/UVA emission power ratio is 0,02 to 0,08.

US 5,557,112 discloses a fluorescent lamp having multiple zones with different ultraviolet radiation characteristics along its length with a first fluorescent coating for producing a first UV radiation and a second fluorescent coating for producing a second UV radiation, which is different from the first UV radiation. Generally, the UVB intensity and UVB/UVA ratio is increased in one longitudinal area. In the example of the suggested lamp, the UVB/UVA ratio of the higher intensity coating is 0,066 and radiation levels are 2,1 (UVA) and 0,14 (UVB) microwatts/cm². The UVB/UVA ratio of the lower intensity coating is 0,052, and the radiation levels are 2,10 (UVA) and 0,12 (UVB) microwatts/cm².

US 4,967,090 offers a fluorescent lamp and system for providing cosmetic tanning with an adjustable UVB/UVA ratio. This adjustment is achieved by two phosphor coatings with different UVB/UVA ratio and an axial rotation of the lamp. The proposed lamp includes a first ultraviolet-emitting phosphor disposed on a portion of the circumference of the interior of an ultraviolet-transmitting glass envelope. A second ultraviolet-emitting phosphor is disposed on the remaining portion the lamp envelope. In one embodiment, the proportions of UVB to UVA from the same light source are about 1,6 % and 4,2 %.

In the prior art tanning lamps the erythemal effect limits the irradiation doses. The tanning effect is achieved by both the UVA and the UVB radiation. UVB radiation must however be limited because of its high erythemal effect. The UVB part of the radiation provides a contribution to vitamin D3 synthesis, however it is not effective enough.

Recently a few UVB lamps have been suggested for medical purposes, such as for the treatment of psoriasis. One group of these lamps provides wide band UVB radiation, while another group may be used for generating narrow band UVB radiation. An example for a wide band UVB is TL/12 and an example for a narrow band UVB is TL/01 available from Philips. These special UVB lamps have no radiation in the UVA and UVC spectral range. The input electric power of the lamps is 100 W and the output radiation level is relatively high, therefore extreme care should be taken in order to avoid overexposure to UVB which may cause sunburn effect (erythema) or may even contribute to developing skin cancer.

Therefore it is an objective of the present invention to provide a fluorescent lamp, preferably a low pressure mercury discharge lamp, that emits an optimized radiation which induces significantly more efficiently the photosynthesis of previtamin D3 in human skin, without exceeding the erythemal effect caused by related prior art products.

### Disclosure of the invention

The objectives of the invention may be accomplished by using a fluorescent lamp, preferably a low-pressure mercury discharge lamp, for stimulating previtamin D3 production. The lamp has a discharge tube with a discharge gas filling. The inside wall of the discharge tube is covered with a phosphor coating for converting short wave UV radiation of the ionized discharge gas into longer wave UV radiation. The discharge tube is closed at both ends and provided with electrodes that are held and lead through a base cap at both ends. The base caps of the lamp have contact pins on them for connecting the lamp to an electrical power supply.

According to the improvement of the invention, the UV light-radiating coating of the discharge tube is comprised of
- a first phosphor for emitting light waves in the UVB spectrum and
- a second phosphor for emitting light waves in the visible light spectrum and for suppressing the emitted light in the UVB spectrum.

The suggested lamp will emit no light in the UVC spectrum, it mainly provides radiation in the UVB spectrum and there may be some radiation in the UVA spectrum, however with less power than in the UVB spectrum. In consequence, this lamp will not provide light waves that cause a useful tanning effect, however it will provide light waves that contribute very effectively the production of previtamin D3 in human skin.

The lamp provides a light emission spectrum with a maximum in the range of 310 nm < λ < 330 nm, preferably within the range of 315 nm < λ < 325 nm. The selected spectral range provides for an effective stimulation of the previtamin D3 production, while the erythemal effect is minimized.

Also, the maximum of the emission spectrum of the lamp is less than 0,05 W/m², preferably less than 0,04 W/m² and even more preferably less than 0,03 W/m². The suggested power range makes it possible to select longer exposure times without negative effects as well as to determine the necessary dosage more precisely.

### Short description of the drawings

The invention will be described in more detail below on the basis of and in connection with exemplary embodiments shown in the drawings, in which
Fig. 1 is a side view of a fluorescent lamp with base caps on both ends also provided with two pairs of contact pins,
Fig. 2 is a cross sectional view of an embodiment of the lamp shown in Fig. 1,
Fig. 3 is a cross sectional view of another embodiment of the lamp shown in Fig. 1,
Fig. 4 is an action spectrum diagram of 7-DHC to previtamin D3 conversion in human skin,
Fig. 5 is a sensitivity diagram of the human skin as a function of the wavelength of the irradiating light,
Fig. 6 is a power spectrum diagram of a conventional mercury vapor sun-tanning lamp combined with the diagram of Fig. 5,
Fig. 7 is the same diagram as shown in Fig 6 completed with a power spectrum diagram of a lamp according to the invention,
Fig. 8 is an enlarged view of Fig. 7, with a range of 290 nm to 330 nm,
Fig. 9 is a power spectrum diagram of the lamps of Fig. 8 weighted with the vitamin D3 sensitivity diagram,
Fig. 10 is a transmission diagram of the glass of the discharge tube of a conventional lamp and the lamp according to the invention.

### Description of preferred embodiments

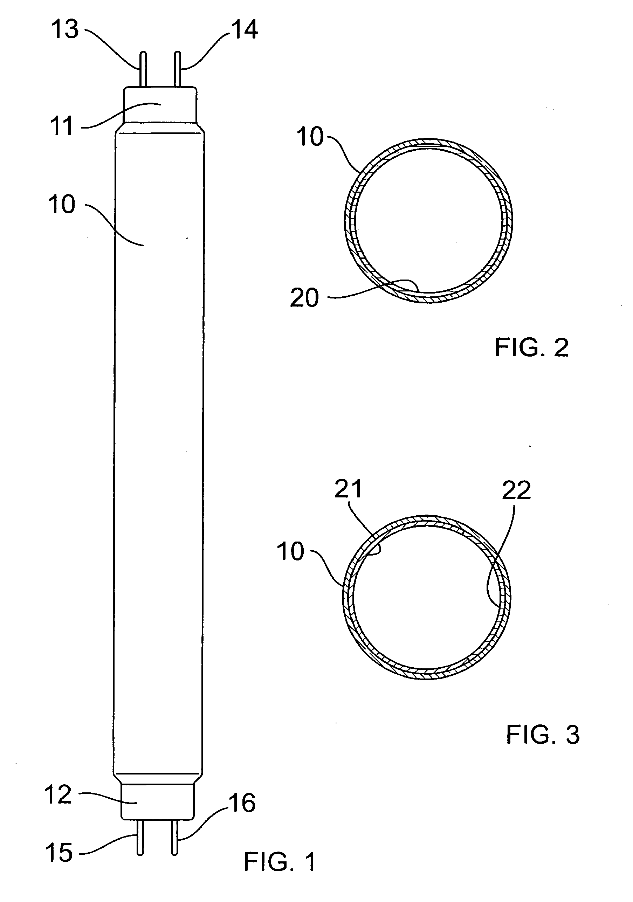

FIG. 1 shows a lamp comprised of a sealed hollow discharge tube 10 made of glass, which contains a quantity of mercury and an inert gas such as argon, krypton or the like. At each end of the glass tube 10 there is an electrode comprised of an oxide-coated tungsten coil and lead-in wires (not shown). Suitable bases 11 and 12 are fixed to the ends of discharge tube 10 and carry contact pins 13, 14 and 15, 16.

The lamp shown in Fig. 1 may be of any standard size, for example 6' of type T12 for use in connection with a sun bed or a smaller sized personal tanning apparatus or of any special size to be used in a special apparatus in order to achieve the desired stimulation of the synthesis of previtamin D3. Although the shown lamp is sealed and has electrodes on both ends of a straight discharge tube, it will be apparent to those skilled in the art that any other form, size, and construction of a fluorescent lamp may be used just as well for the purposes of the invention.

In a fluorescent lamp usually a suitable phosphor coating is used on the inner surface of the discharge tube which will absorb the shorter wave UV radiation (mainly UVC radiation) generated by the arc discharge within the lamp and re-emit this energy at a different, more useful longer wave UV spectrum.

According to the invention a mixture of at least two phosphors is used for creating a suspension which is used for covering the inside surface of the discharge tube. This coating is then burned to provide the required mechanical strength. As it is apparent to those skilled in the art, further layers may be applied in addition to the phosphor layer. Such layers may for example include a protective, a reflective layer and other layers. The two component phosphor layer provides a light emission mainly in the UVB spectrum and a visible spectrum. One phosphor of the mixture is responsible for the UVB radiation and the other phosphor provides for a visible light emission and a suppression of the UVB radiation. Different phosphors may be used as a UVB phosphor. One such phosphor may be a cerium-activated magnesium barium aluminate ((Mg,Ba)Al₁₁O₁₉:Ce) phosphor. It might be of further advantage if the second phosphor is a phosphor for emitting visible yellow light. On one hand, it improves the aesthetic effect of the lamp in use. On the other hand, it has been found especially suitable for suppressing the light emission power in the UVB range. Different phosphors may be used for providing visible yellow light. One such phosphor may be cerium-activated yttrium aluminate (Y₃Al₅O₁₂:Ce) phosphor. The saturated yellow color of the Y₃Al₅O₁₂:Ce phosphor has the additional benefit of resulting in a nice aesthetic appearance that gives the impression that the client has been sunbathing.

Regarding another aspect of the invention, the proportion of the second phosphor to the first phosphor is selected to be in the range of 30 wt% and 50 wt%. On one side, a lower proportion (below 30 wt%) of the yellow phosphor would not provide sufficient visible light and suppression in the UVB spectrum. On the other hand, however, a higher proportion (above 50 wt%) of the yellow phosphor would result in two much energy of the visible light and an undesired level of suppression in the UVB spectrum.

In order to achieve a uniform effect, the two phosphors 20 in a suspension may be distributed uniformly on the inner surface of the envelope of the discharge tube 10 as shown if Fig. 2, where the discharge tube 10 has a uniform phosphor layer 20 on the inside surface.

According to another embodiment, as shown in Fig. 3, the discharge tube 10 has a first phosphor layer 21 on a part of the inside surface of the discharge tube 10 and a second phosphor layer 22 on the remaining part of the inside surface of the discharge tube 10. Such an embodiment may be advantageous if different effects along the surface of the lamp should be achieved. To this effect, any pattern may be used for an uneven distribution along the inner surface of the discharge tube to provide for the desired uneven effect.

According to one aspect of the invention, the two phosphors may have an uneven distribution along the longitudinal direction of the discharge tube. This would enable to provide different power spectral distribution characteristics of the lamp along the longitudinal direction, and therefore different exposure of different parts of the human body. In another aspect of the invention, the two phosphors may have an uneven distribution along the circumferential direction of the discharge tube. This would provide a lamp with different power spectral distribution characteristics of the lamp along the circumferential direction. Such a lamp could be used for example as a combination of a conventional sun tanning lamp and a lamp for stimulating the synthesis of previtamind D. As it may be apparent to those skilled in the art, different additional phosphors and layers may be applied in order to achieve different additional effects.

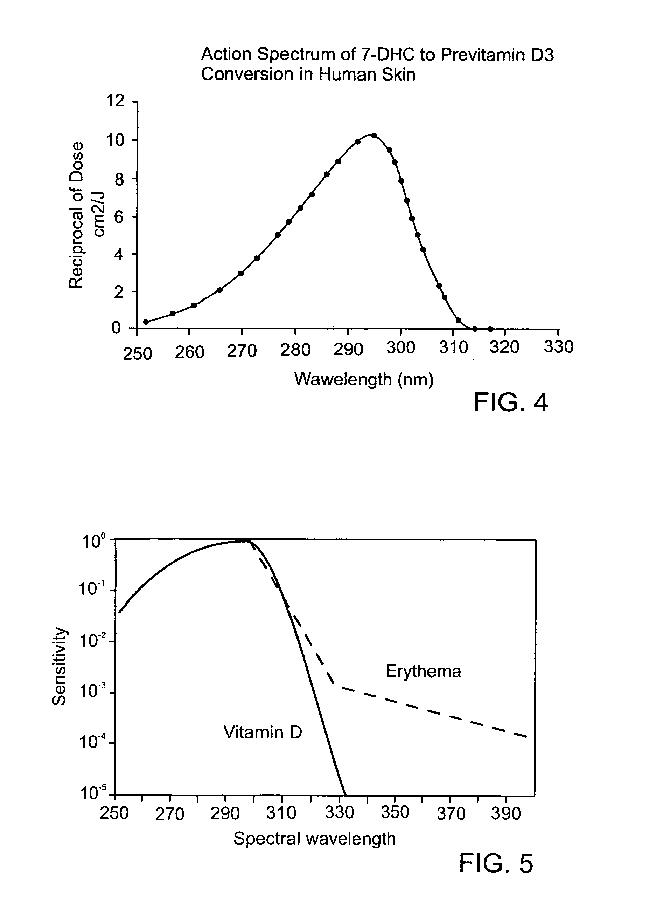

In Fig. 4, the action spectrum of 7-DHC (7-dehydrocholesterol) to previtamin D3 conversion in human skin can be seen in terms of the reciprocal dose (cm2/J) depending on the wavelength of the light. This diagram represents the relative sensitivity of human skin and was publised by MacLaughlin J.A., Anderson R.R., Holick M.F. in Spectral character of sunlight modulates photosynthesis of previtamin D3 and its photo isomers in human skin. (Science 216(4549):1001-1003, 1982). The photosynthesis of previtamin D3 from 7-dehydrocholesterol in human skin was determined after exposure to narrow-band radiation. The optimum wavelengths for the production of previtamin D3 were determined to be between 295 and 300 nanometers.

According to a CIE (International Commission on Illumination) technical report (CIE 174:2006) the action spectrum for the production of previtamin D3 in human skin has a maximum at about 300 nm and no significant action can be found above 330 nm. According to the relative sensitivity diagram shown in Fig. 5, a spectrum between 250 nm and 320 nm may be used for stimulating previtamin D3 synthesis. There is, however, another effect caused by UV radiation in human skin that has to be taken into consideration. If human skin is exposed to shorter wave UV light, especially UVB and UVC, there may be an undesired sunburn effect. The relative sensitivity of skin to develop sunburn effect, or erythema, is shown in a second diagram with a broken line in Fig. 5. As it can be seen clearly, the relative sensitivity to erythema is very high in the range of λ < 300 nm. The highest sensitivity is determined to be 100% or 1. This sensitivity drops very rapidly from 10° to 10⁻³ in the range of 300 nm < λ < 330 nm and drops less rapidly from 10⁻³ to 10⁻⁴ in the range of λ < 330 nm. Conventional sun tanning lamps emit light in the UVA range and practically no or very few light in the UVB range causing minimal erythemal effect.

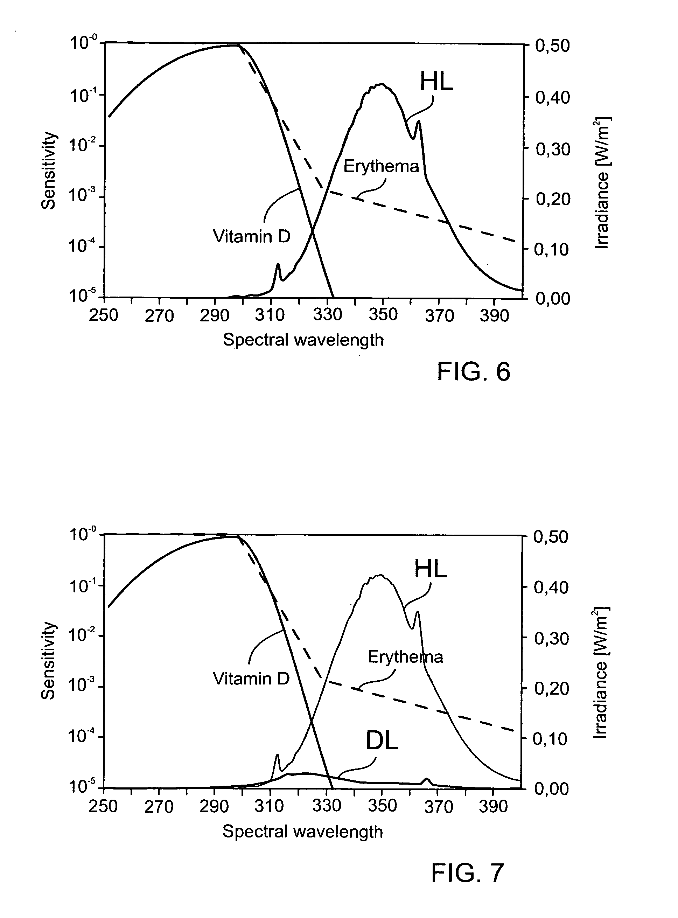

Fig. 6 shows the emission power spectrum of a conventional sun-tanning lamp HL such as those produced by Philips under the trade name Swift 100R (also known as Dr Holick UV-System Vitamin D3 Sunlamp) and the relative sensitivity diagrams (previtamin D3 and erythema action spectra) of the skin. All irradiance data is measured at 25 cm distance from the middle of the lamp, according to IEC 61228. It consumes 100Wrms electric power and emits 17,7 W/m² total UVA radiation and 0,33 W/m² total UVB radiation. The total radiation weighted by the previtamin D3 action spectrum is 44,6 mW/m². The total radiation weighted by the erythema action spectrum is 58 mW/m², which results in approximately 8 minutes exposure, depending on the sun bed or sun booth in which they are applied, to reach a minimum erythemal dose (one MED) when exposed to one lamp. A significant part of the total erythema is obtained from the UVA part of the lamp spectrum, instead of the UVB range, in an approximate 25:75 ratio. This lamp has a power spectrum substantially in the UVA range with two local peaks at 311 nm and 365 nm due to the mercury arc. The maximum emission power (irradiance) of about 0,42 W/m² can be observed at a wavelength of 365 nm. The irradiance values for the power spectrum diagram can be seen on the right side in the range of 0 to 0,5 W/m². The relative sensitivity values are shown on the left side. This prior art sun-tanning lamp has a good tanning effect in the UVA range of 330 nm < λ < 370 nm with little erythemal effect, and also contributes to vitamin D synthesis in the UVB range of 300 nm < λ < 330 nm, especially at the 311 nm peak of the mercury arc. The UVB range however poses a high risk of erythema which further limits the allowable exposure time.

The lamp also has a certain stimulating effect for previtamin D3 production but because of the high erythemal effect no sufficient exposure time may be selected in order to achieve an effective previtamin D3 synthesis.

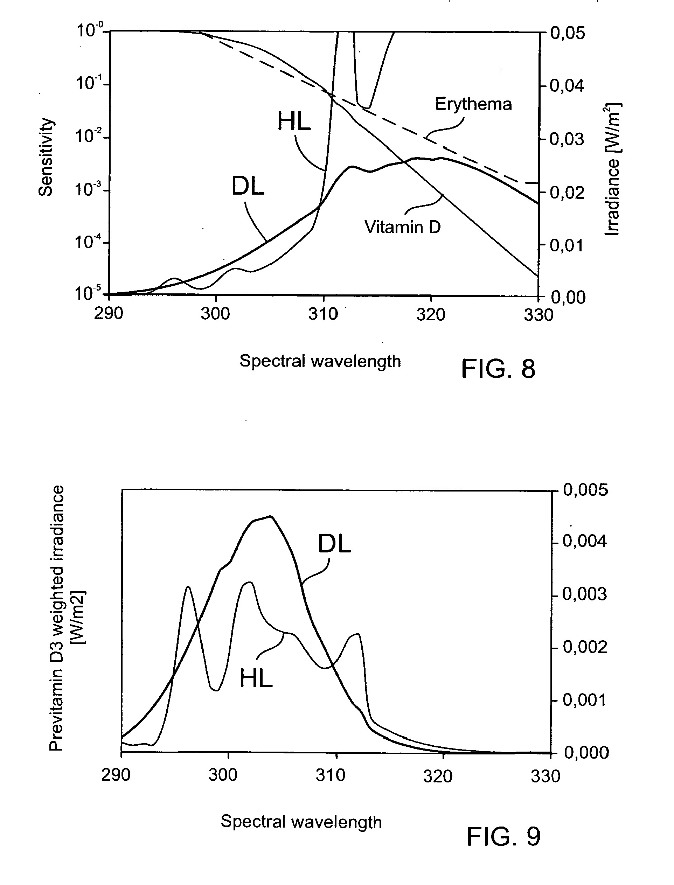

Figs. 7 and 8 show the power spectrum diagram of a lamp DL according to the invention in comparison with a conventional lamp HL shown in Fig. 6 and with the relative sensitivity diagrams of the skin. Fig. 7 shows the same diagrams as already seen in Fig. 6 with a new power spectrum diagram of a lamp DL according to the invention. Fig. 8 shows these diagrams in an enlarged view with a smaller spectral and irradiance range. As best seen in Fig. 7, the lamp according to the invention provides a light emission spectrum mainly in the range from 290 nm to 330 nm, preferably in the range of 315 nm and 325 nm with a maximum at about 325 nm. The proposed lamp has no significant emission in the range below 290 nm and above 340 nm. The selected spectral range provides for an effective stimulation of the previtamin D3 production, while the erythemal effect is minimized.

The maximum of the emission spectrum of the lamp according to the invention is less than 0,05 W/m², preferably less than 0,04 W/m² and even more preferably less than 0,03 W/m². As shown in Fig. 8 in connection with an exemplary embodiment of the invention, the maximum power (0,025 W/m²) of irradiance of the new lamp is significantly lower than the peak power (0,06 W/m²) of the prior art lamp in the UVB range. On the other hand, the new lamp has a higher output power in the range of 290 nm < λ < 310 nm which results in a better stimulating effect for producing previtamin D3. The suggested power range makes it possible to select longer exposure times without negative effects and to determine the necessary dosage more precisely. As it can be seen best in Fig 8, the lamp DL has a significantly higher emission in the range of 295 nm to 310 nm resulting in a better stimulating effect for the production of previtamind D3.

The effectiveness of the stimulation of the previtamin D3 synthesis is best seen in Fig. 9, which shows the power spectra of the lamp according to the invention and the conventional sun tanning lamp weighted with the previtamin D3 action spectrum (relative sensitivity of the skin). The weighted diagram of the lamp according to the invention peaks at about 305 nm and as it can be clearly seen, in most of the active range of the power spectrum of the lamp according to the invention, the output power is higher than that of the conventional lamp HL. In consequence, the suggested lamp DL has a significantly better previtamin D3 efficacy at the same or lower erythemal effect.

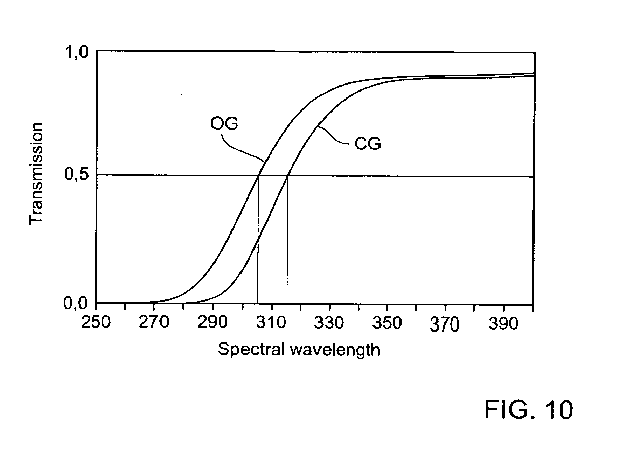

In Fig. 10, the transparency diagrams of two different glasses are shown in a comparable way. As seen on the figure, both of the glasses are transparent to light with a wavelength of λ > 350 nm and opaque to light with a wavelength of λ < 280 nm that is for the UVC spectrum. Both of the glasses have a similar transparency diagram, which is offset along the x axis by about 10 nm. The 50 % transparency of the first glass OG is at λ = 305 nm and the 50 % transparency of the second glass CG is at λ = 315 nm. Since the first glass is transparent for shorter waves, it is also referred to as "open glass"; the second glass, which is not transparent to these shorter waves, is also referred to as "closed glass" in the sun tanning industry.

In order to further reduce the intensity of the emitted UVB spectrum of the lamp according to the invention, it might be advantageous to use a "closed glass" instead of an "open glass". The discharge tube therefore may be made of a glass material which has a 10 % transparency for the light waves with a wavelength of k > 295 nm. According to a further aspect, the glass material of the discharge tube may have a 50% transparency for the light waves with a wavelength of λ > 310 nm, preferably of λ > 315 nm. In order to achieve the so called "closed" characteristic of the glass, additives such as Fe, Ce or Ti may be added to the glass material resulting in a slightly different transparency curve which makes it necessary to reoptimize the phosphor blend.

In the example shown in Fig. 10, the first glass OG was a glass LT 101 (open glass) available from LightTech, Budapest. The second glass CG was a glass LT 103 (closed glass) available from LightTech, Budapest.

In order to further decrease the emitted light power, the input electric power of the lamp may be selected in a low wattage range of about 40W so that the lamp can be driven at lower arc current, preferably with a 40W electromagnetic ballast. This has the additional benefit of consuming 60% less electric power, which enables a less expensive usage of the device during its functional lifetime. Lower wattage electromagnetic ballast cannot operate 6' lamps stably due to the high arc voltage. A specially designed electronic ballast would be able to operate a 6' T12 lamp in a stable way, but such ballast is not favored due to its high cost compared to an electromagnetic ballast.

The following table provides a comparison of a prior art lamp and an example according to the invention. For the purposes of a comparative test sample we used a 6' long discharge tube made of a closed glass without a reflecting layer, with a short mount for holding the electrodes, with a phosphor blend of 65 wt% UVB phosphor of the type NP807-32, 35 wt% yellow phosphor of the type NP204 both available from Nichia (Tokushima), an inert gas filling of 50 wt% krypton and 50 wt% argon at a cold filling pressure of about 2 mbar. The low wattage (40 W) power supply and the electromagnetic ballast provided a cathode current of about 1A.

**Table 1**

| | Dr. Holick Vitamind D3 Sunlamp | Example according to the invention |
|---|---|---|
| Lamp power (W) | 100 | 40 |
| Total UVA (W/m²) | 17,7 | 0,8 |
| Total UVB (W/m²) | 0,33 | 0,26 |
| Erythema (mW/m²) | 58,1 | 49,3 |
| Erythema A (mW/m²) | 14,9 | 1,1 |
| Erythema B (mW/m²) | 43,2 | 48,2 |
| Previtamin D3 (mW/m²) | 44,6 | 57,4 |
| Previtamin D3 efficacy (mW/m2/W) | 0,47 | 1,49 |

The lamp according to the invention has a high efficiency in converting electric power to previtamin D3 production when compared to the prior art lamp by Dr. Holick. Both the total UVA and total UVB radiation of the tested example are lower than that of the prior art lamp, but it has as high as 58 mW/m² previtamin D3 weighted irradiance even when operated with a low power 40W conventional electromagnetic ballast, while its erythema is 49 mW/m², resulting in 8.5 min MED for one lamp. This means that using this lamp the client gets 30% higher previtamin D3 dose during the same exposure session, without an increased risk of sunburn. In addition, the previtamin D3 efficacy (calculated as dividing the previtamin D3 weighted irradiance with the lamp wattage, similar to the "lumen per watt efficacy" of general lighting lamps) is three times higher than that of the prior art lamp. (See Fig 9 for spectral comparison and Table 1 for data.)

The fluorescent lamp according to the invention has a significantly higher vitamin D3 efficacy, because it induces more previtamin D3 production in human skin during the same exposure time, without causing more skin burn.

## Claims

1. A fluorescent lamp, preferably a low pressure mercury discharge lamp for stimulating D3 previtamin production comprising a discharge tube with a discharge gas filling, the discharge tube being covered on the inside wall with a phosphor coating for converting short wave UV radiation of the ionized discharge gas into longer wave UV radiation, the discharge tube further being closed at both ends and being provided with electrodes which are held and lead through base caps which are provided with contact pins for being connected with an electrical power supply, **characterized in that** the UV light radiating phosphor coating of the discharge tube comprises a mixture of at least two phosphors with
- a first phosphor for emitting light waves in the UVB spectrum and
- a second phosphor for emitting light waves in the visible light spectrum which suppresses the emitted light in the UVB spectrum.

2. The fluorescent lamp of claim 1, **characterized in that** the light emission spectrum has a maximum in the range of 310 nm and 330 nm, preferably in the range of 315 nm and 325 nm.

3. The fluorescent lamp of claim 1, **characterized in that** the maximum of the emission spectrum is less than 0,05 W/m², preferably less than 0,04 W/m² and even more preferably less than 0,03 W/m².

4. The fluorescent lamp of claim 1, **characterized in that** the first phosphor comprises (Mg,Ba)Al₁₁O₁₉:Ce.

5. The fluorescent lamp of claim 1, **characterized in that** the second phosphor is a phosphor for emitting visible yellow light.

6. The fluorescent lamp of claim 5, **characterized in that** the second phosphor is Y₃Al₅O₁₂:Ce.

7. The fluorescent lamp of claim 6, **characterized in that** the proportion of the second phosphor to the first phosphor is at least 30 wt% and not more than 50 wt%.

8. The fluorescent lamp of claim 7, **characterized in that** the two phosphors are distributed uniformly on the inner surface of the envelope of the discharge tube.

9. The fluorescent lamp of claim 7, **characterized in that** the two phosphors have an uneven distribution along the inner surface of the discharge tube.

10. The fluorescent lamp of claim 9, **characterized in that** the two phosphors have an uneven distribution along the longitudinal direction of the discharge tube.

11. The fluorescent lamp of claim 9, **characterized in that** the two phosphors have an uneven distribution along the circumferential direction of the discharge tube.

12. The fluorescent lamp of claim 1, **characterized in that** the discharge tube is made of a glass material which has a 10 % transparency for the light waves with a wavelength of λ > 295 nm.

13. The fluorescent lamp of claim 12, **characterized in that** the glass material of the discharge tube has a 50% transparency for the light waves with a wavelength of λ > 310 nm, preferably of λ > 315 nm.

14. The fluorescent lamp of claim 1, **characterized in that** the lamp is a low wattage lamp with about 40W.
